# EUROPEAN PATENT APPLICATION

(11) **EP 3 363 861 A2**
(43) Date of publication of application: **22.08.2018**
(21) Application number: 16855814.6
(22) Date of filing: 11.10.2016
(51) Int. Cl.: C08L 29/04, C08L 5/06, A61K 47/32, A61K 47/36

(54) **POLYMER COMPOSITION**

(30) Priority: 15.10.2015 MX 2015014524
(71) Applicant: Centro De Investigación Y Asistencia En Tecnología Y Diseno Del Estado De Jalisco, A.C. (CIATEJ, A.C.), 44270 Guadalajara, Jalisco (MX)
(72) Inventor: GARCÍA CARVAJAL, Zaira Yunuén, Guadalajara Jalisco 44270 (MX); ESPINOSA ANDREWS, Hugo, Guadalajara Jalisco 44270 (MX); GONZÁLEZ ÁVILA, Marisela, Guadalajara Jalisco 44270 (MX); HERRERA RODRÍGUEZ, Sara Elisa, Guadalajara Jalisco, 44270 (MX); HERNÁNDEZ GUTIÉRREZ, Rodolfo, Guadalajara Jalisco 44270 (MX); RODRÍGUEZ RODRÍGUEZ, Rogelio, Zapopan Jalisco 45130 (MX); TORRES ROSAS, Marco Eduardo Andrés, Guadalajara Jalisco 44270 (MX); FLETES VARGAS, Ana Gabriela, Guadalajara Jalisco 44270 (MX)
(74) Representative: Regimbeau
(86) International application number: PCT/MX2016/000108
(87) International publication number: WO 2017/065597

(57) **Abstract**

In the present invention, gelled polymeric compositions for controlled release are described, which admit the incorporation of a range of chemical, pharmaceutical or food substances, in order to be a transport towards the gastrointestinal tract. The gelled polymeric compositions as described in the invention are used as the basis for the preparation of, for example: excipients, vehicles, coatings, films, hydrogels, among others, thus being highly profitable for the development of products for the pharmaceutical industry and/or the food industry.

## Description

### TECHNICAL FIELD OF THE INVENTION

The present invention has its field of application on the biotechnological area, particularly on the development of products for the pharmaceutical industry and the food industry.

### BACKGROUND OF THE INVENTION

Polyvinyl alcohol (PVA) is a water soluble synthetic polymer; unlike most polymers derived from vinyls, PVA is not prepared by polymerization of the corresponding monomer, vinyl alcohol. Instead, it is prepared by partial or complete hydrolysis of the poly (vinyl acetate). The reaction with the ethyl group can be controlled and the physical characteristics of the resulting PVA will depend both on the polymerization grade and the hydrolysis grade of the starting poly (vinyl acetate). Its water solubility is limited and is considered optimum between 87 and 89 % of ethyl group hydrolysis. In the most advanced hydrolysis, PVA presents a high tendency to form hydrogen bonds association, which easily leads to gels formation. PVA is already used in areas such as food chemistry, pharmaceutical products, medicine and biotechnology. In particular, PVA is a polymer of great interest for various pharmaceutical and biomedical applications. PVA-based microspheres have already been approved by the Food and Drug Administration (FDA) and other regulating organisms for embolization. Moreover, PVA has excellent adhesive, film formation; hydrogel, cryogel and emulsifier formation properties. Additionally, depending on the type of additives they could contain, they could be regarded as biocompatible and non-irritating for soft tissues upon making contact with them, making them adequate for many biomedical applications (Marc Chaouat, Catherine Le Visage, Wilms E. Baille, Brigitte Escoubet, Frédéric Chaubet, Mircea Alexandru Mateescu, and Didier Letourneur., 2008). Several methods have been described for the preparation of PVA-based materials. For example, high performance PVA films can be prepared by molding in the presence of an adequate plasticizer. Physical reticulation of PVA has also been performed by freezing and thawing cycles, with formation of rather soft hydrogels (Gutiérrez, M. C., Garcia-Carvajal, Z. Y., Jobbágy, M., Rubio, F., Yuste, L., Rojo, F., Ferrer, M. L. and Del Monte, F., 2007).

On the other hand, pectin is a polysaccharide constituted by D-galacturonic acid units attached by bonds (α 1-4) with ramification points composed of rhamnose and arabinose residues. Pectin has been applied on the synthesis of polymeric matrices for controlled drug release, protein and cell immobilization (Seixas F. L., Fukuda D. L., Turbiani F. R. B., *et al*., 2014; Wikiera A., Mika M., Starzyríska-Janiszewska A., Stodolak B., 2015). Pectin is classified among soluble fibers, which are resistant to hydrolysis of digestive enzymes; however, enzymes produced by rumen microorganisms cause pectin fragmentation in ruminants; which allows its application as an interesting alternative for a ruminal supply system, since this natural polymer remains intact until reaching the ruminal environment (Cruz M., Fernandes K., Cysneiros C., Nassar R., and Caramori S., 2015). Pectin has been employed in multiple food industry applications, since it is a gelling and thickening agent with stabilizing properties. Pectins are used in the production of jams, fruit jellies, acidified milk, protein drinks, yogurts and other dairy products, among others. Nowadays pectin is also applied on the non-food industry: such as medicine and pharmaceutical industries (Lewandowska K., D 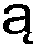 browska A., Kaczmarek H., 2012).

Physical-chemical properties of both pectin and PVA can be modified in several ways. A simple way is simply mixing with other macromolecular compounds at different concentrations. This will condition that the mixture at different proportions and concentrations has a not-so-obvious effect on applications for which it will be required (Lewandowska K., D 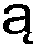 browska A., Kaczmarek H., 2012).

In the document Lewandowska, K., *et al* 2012, the rheological properties of Pectin-PVA mixtures were studied. Several mixtures were prepared at ratios of 70:30, 50:50, 30:70 (Pectin:PVA). PVA concentration was 7.8 % w/v and for Pectin it was 3 % w/v. The PVA employed has a molecular weight of 30,000-70,000 g/mol and pectin has a 27.8 % of esterification. The obtained results demonstrated that flux properties of the mixtures solutions depend on their composition, temperature and shearing rate, since there are conformational changes of macromolecules because of chemical interactions. In spite of this, this document does not demonstrate an application for the industry.

In the document Kaczmarek, H., *et al.,* 2011, Pectin-PVA films were prepared at different ratios and physical-chemical interactions were studied by infrared spectroscopy on the films. The starting polymeric composition consisted of 2 % Pectin w/v and 2 % PVA w/v (98 % of hydrolysis, average molecular weight of 85,000-146,000 g/mol). The mixtures ratios for film preparation were 70:30, 50:50 and 30:70, Pectin:PVA. This research work demonstrated that the Pectin-PVA mixture is capable of forming films and that despite having the same polymer concentration and different ratios thereof, some intermolecular interactions appear (hydrogen bonds, dipole-dipole type) between PVA-Pectin macromolecules, which modify the mechanical properties (in this case, flexibility) thereof. These properties will depend on many factors, such as: chemical composition of the sample, accessibility and conformational disposition of the functional groups, steric impediments, etc. Being these interactions crucial for the improvement of mechanical properties; showing again that the composition and ratio of this mixture play a critical role. However, this research work does not demonstrate the impact the composition has on industrial functionality (Kaczmarek, H., D 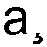 rowska, A. y Vukovic-Kwiatkowska, I., 2011).

In the document Martinez YN, Piñuel L, Castro GR, Breccia JD Martinez YN, 2012, it was demonstrated that the Pectin-PVA polymeric mixture is capable of forming film-type cryogels for use in an antibiotic release system (enrofloxacin). This system contained 15 % PVA w/w, 0.1 and 1.0 % pectin w/w and an enrofloxacin load of 5-35 µg. To evaluate the impact of cryogels on the enrofloxacin release profile, these were placed on a diffusion manual system, suggesting its application as a topical (dermal) release system. In the shown results it is observed that the 100 % release of encapsulated enrofloxacin was reached during the first hours of experimentation. This work shows the utility of this mixture in the form of cryogels as a topical controlled release system of bioactive substances. It is worth mentioning that the polymeric mixture composition described in this document does not correspond to the one proposed in the present invention.

WO 2014/014348 A1 refers to the invention of a floating drug administration system. Ideally, these systems should have a prolonged gastric permanence time; however, this is not always the case. This invention refers to the coating of the floating system, which comprises a polymer selected from the group consisting of hydrophilic cellulose derivatives, such as HPMC, HPC, MC, HEC, CMC, sodium-CMC, PVP, PVA, carboxyvinyl polymer (carbomer), poly (ethylene oxide) (Polyox WSR), alginates, pectins, guar gum, vinylpyrrolidone-vinyl acetate copolymer, dextrans, carragenin, gellan, hyaluronic acid, pullulan, scleroglucan, xanthan, and xyloglucan. Moreover, it comprises one or more coating layers which comprise a combination of HPMC and starch as a coating material in a ratio within the range of 8:1 - 1:1. Despite the fact that this formulation comprises PVA and pectin, neither the composition nor the ratio thereof is indicated in said patent document.

US 2004 0176535 A1 refers to a polymeric composition based on PVA, which is substantively composed of 50 to 99.9 % PVA w/w, with an average molecular weight of 5 000 to 25 000 and with a hydrolysis grade of 79 to 99.9 %. 2 examples in which a pectin-PVA mixture with different emulsifiers is included are described. Viscosity was also measured. Additionally, comparative examples where pectin is not present in the composition are included, with the purpose of comparing the effect pectin has on the viscosity of the obtained suspensions. Both pectin and PVA were added at different ratios (the PVA employed in US 2004 0176535 A1 is different from the one in the proposed invention). As expected, viscosity of the polymeric compositions is different, showing again that the ratios of these two polymers play an important role in the physical-chemical and mechanical properties.

WO 1997011113 A1 describes the preparation of flexible films starting from a pectin-PVA mixture as a potential plasticizer. This combination is advantageous since it increases biodegradability. In this case, pectin has a high molecular weight, great turning radius, it has a high grade of methyl esterification and it has high intrinsic viscosity. The PVA employed has a molecular weight of 124,000-186,000 with a 99 % hydrolysis grade and between 10 to 90 % w/v. Additionally, the use of pectin provides the effective utilization of an agricultural product.

WO 2002017886 A1 refers to the composition of a film comprising a) pectin, b) a second film-forming polymer and c) an adjustment system. The second film-forming polymer is selected from gelatin, pullulan, polyvinyl alcohol, hydroxypropylated starch, hydroxyethylated starch, hydroxypropyl methylcellulose, hydroxypropyl cellulose, methyl cellulose, hydroxyethyl cellulose, hydroxyethyl methyl cellulose, or mixtures thereof. The pectin content employed was from 5 to 50 %, but it is preferred from 10 to 40 %, and the content of the second polymer ranges between 60 and 95 %, but preferably from 50 to 85 %. This fixing system is intended for use in pharmaceutical, veterinary, food, cosmetics or other products such as food wrapping films, gelatins or jellies, preferably for pre-dosed formulations such as soft or hard capsules, as well as aqueous solutions of the compositions for the manufacture of said products. Although the compositions described herein could be similar to those proposed in the invention, it should be emphasized that the Pectin-PVA ratios disclosed in WO 2002017886 A1 are far from those concerning this invention.

WO 2006/122835 A1 describes oral administration devices for the colonic release of adsorbents, comprising: a) an adsorbent capable of adsorbing antibiotics, a bacterial or fungal toxin, or a pharmaceutically active agent that can cause adverse side effects when they reach the colon, and b) a drug delivery device based on pectin beads, which can be crosslinked with metallic ions such as Ca and/or Zn. The delivery system protects the adsorbent and prevents its adsorption effect in the upper gastro-intestinal tract. When the particles reach the colon, proteolytic enzymes degrade pectin, enabling the adsorbent to be released and exert its activity. In this system, the crosslinked pectin contains polyethyleneimine (as a crosslinking agent); in addition, the starting point is a solution comprising 1-10 % (w/v) of pectin and 2-12 % (w/v) of zinc acetate or calcium chloride. The system proposed in this invention has the advantage over what is disclosed in WO 2006/122835 A1, that with the optimal proportions of PVA-Pectin the expected effects are achieved without requiring chemical cross-linkers.

In CN102343054 A, the invention provides the preparation and use of a film type coating. Tablets that promote digestion were coated. This formulation contains an energetic (Jianweixiaoshi leaf). This invention belongs to the technical field of medicinal production: when hydroxypropylmethylcellulose is used as a pharmaceutical coating of tablets, it is easy for the tablets to stick together with one another causing cracks and bulges in the coatings and this has the consequence that during the chewing process of the tablets, the films are taken off from the tablets and the sensation in the mouth of the product is influenced. The film-type coating mainly comprises lactose, pectin and polyethylene glycol 4000.

Both PVA and pectin are accepted as food additives. PVA has been accepted as a film coating agent for food supplements, in particular in applications where protection against moisture is required; while pectin is accepted as a stabilizing agent (Official Journal of the European Union. COMMISSION DIRECTIVE 2010/69/EU of 22 October 2010. Amending the Annexes to European Parliament and Council Directive 95/2/EC on food additives other than colors and sweeteners).

It has recently been shown that pectin and its mixtures plasticized by glycerol can be extruded successfully for the formation of films with good mechanical properties. The complex structure of the pectin gel aggregates has been researched in detail by atomic force microscopy.

Therefore, the relevance in determining the behavior and properties of polymers to make better use of them is shown. This objective is achievable, according to the invention, without the application of very sophisticated methods, often inaccessible and expensive, simply with a base in the study of the rheological properties of the polymers and their combinations. Flow properties of the mixture solutions were used depending on their composition, temperature and shear rate among others.

### BRIEF DESCRIPTION OF THE FIGURES

Figure 1. It shows the treatments used in the different systems: Number of coating layers applied to the system: 0 and 2; and Thermal treatment: A) not subjected to a freezing/thawing cycle; B) subjected to a freezing/thawing cycle.
Figure 2. It shows: A) systems coated with a PVA/Pectin film with freezing/thawing cycle, B) systems coated with PVA/Pectin film without freezing/thawing cycle, C) uncoated systems.
Figure 3. A) The solubility enabled by the PVA/Pectin film in the different systems and different treatments is observed, B) close up of treatment B, where the PVA/Pectin film still adhered to the systems is observed.
Figure 4. Systems treated with PVA/pectin after 20 min in the gastrointestinal *ex vivo* simulator are observed.

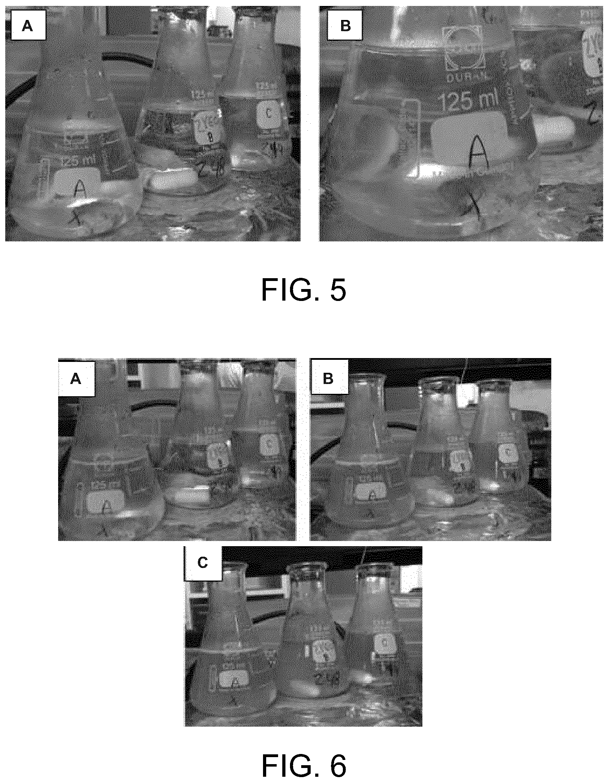

Figure 5. Photographs showing: A) the system with treatment A, the PVA/Pectin film failed to adhere adequately, B) close up of system A, the PVA/Pectin detached film is observed.
Figure 6. The solubility of the systems at different times of study in the gastrointestinal simulator is shown: A) after 4 min, B) after 9 min and, C) after 20 min.
Figure 7. Criteria followed for the evaluation of the release profile of the carmine red dye in the hydrogels are shown: A) the dye was deposited in the hydrogel in amounts of 25, 50 and 100 mg. B) the dye was combined with the PVA-Pectin formulation in amounts of 20 mg.
Figure 8. The release kinetics of the dye incorporated in the PVA-Pectin formulation during hydrogel preparation is shown. The first two hours of evaluation correspond to the Stomach simulator, while the remaining four hours correspond to the Intestine simulator.
Figure 9. The release kinetics of the dye deposited in the cube-shaped hydrogels is shown. The release of dye in the hydrogel occurs in a great amount after 20 hours in simulation, in the transverse colon section.

### DETAILED DESCRIPTION OF THE INVENTION

The present invention relates to gelled polymeric compositions that admit the incorporation of different substances with the purpose of being a transport to the intestinal system, allowing a release thereof in the colon or somewhere else along the gastrointestinal tract. Within the variety of substances that can be incorporated into the gelled polymeric compositions according to the invention are included, for example: chemical compounds, pharmaceutical substances, bioactive substances, nutrients, food additives, dyes, gels, capsules, granules, spheres, particles, nanoparticles, the individual or combined incorporation of these or other substances being possible.

The polymeric composition according to the invention provides a controlled release means that allows eliminating or reducing unexpected effects, for example, that the released substance exceeds the therapeutic or nutritional requirements causing toxicity, or that it is effective when not delivered at the required doses.

Additionally, the controlled release means that is provided with the present invention allows targeted release to specific locations, ensuring that the substance(s) of interest is(are) released at the required location, maintaining its integrity up to that moment.

Thus, an object of the invention is to provide a gelled polymeric composition to be used as a basis for the preparation of, for example: a controlled pharmaceutical release system, an excipient included in some pharmaceutical form, a vehicle for releasing active ingredients that could have functional elements in the food and confectionery industry. Or, to be used, for example, as an edible coating, transparent or with added color.

According to the invention, the polymeric composition contains a natural polymer that is selected from the group of polysaccharides such as: pectin, fructans, chitosan, chitin, celluloses, starches, modified starches, amyloses, amylopectins, mannans, galactomannans, and arabinocylanes. And it also contains a synthetic water-soluble polymer selected from the group of: polyethylene glycol, polyvinyl alcohol, polyvinyl pyrrolidone, polyacrylic acid, and polyphosphates. The polymeric composition according to the invention improves the mechanical properties of the gels, which will be used as vehicles, films, coatings, gels, hydrogels and/or polymeric conjugates, as it is a biocompatible, biodegradable and non-toxic composition.

The polymeric composition of the invention, when in a gelled form, supports acid pHs, making it possible to have formulations with a potential application as carrying systems for bioactive substances, drugs, nutrients, among others. As well as potential polymeric coatings for their protection. The complete composition allows the incorporation of active ingredients for its potential release in any part of the gastrointestinal system being very useful in the food industry and/or in the pharmaceutical industry.

In a preferred embodiment of the invention, the polymeric composition is formed from a natural polymer such as pectin, present in a percentage ranging from 1 to 6 %; and a synthetic polymer such as polyvinyl alcohol with a molecular weight of 89,000 to 98,000 KDa, and which is present in a percentage ranging from 5 to 15 %.

### BEST METHOD FOR REALIZING THE INVENTION

In order to allow a better understanding of the invention, details of some aspects thereof are provided, as well as the non-limiting examples described below.

### EXAMPLE 1

### Polymeric composition of PVA/Pectin

A mixture of polymers PVA (7.8 %) and pectin (5%) was made. First, the PVA was added little by little in 100 ml of water at 80 °C, stirring until completely dispersed. Later, the citrus pectin was added slowly, in order to avoid the formation of lumps. The gel formed was allowed to cool for later use.

Through the use of an *ex vivo* gastrointestinal simulator, the determination of the erosion time of a coating made with the polymeric composition according to the invention was carried out, and arranged on different test systems consisting of edible products such as: pharmaceutical forms presented as tablets, candies and chocolates.

The erosion was performed in an acid medium simulating stomach conditions. Details of the methodology used for the formulation of the polymeric films and/or coatings made with the polymeric composition according to the invention, as well as the tests in the digestive tract simulator are detailed below.

### Coating of the test systems

a. Products of the pharmaceutical industry: commercially available tablets.
b. Coating on products of the food industry: gumpastes also known as gummies or jellybeans, commercially available.
c. Coating over products of the food industry: commercially available bar chocolate.

Using a brush, 0 and 2 layers of coating were made to each system, a part of them underwent the freezing/thawing process and another part was only allowed to dry at room temperature. Both processes were left to rest for 48 hours.

Once the films of each system were obtained, they were submitted to the gastrointestinal simulator. The gastrointestinal simulator consisted of a solution of distilled water adjusted to a pH of 2.50 at a temperature of 37 °C and in constant stirring for 2 h.

Results showed a low index of adhesion of the films in each of the systems, probably due to a low porosity index and a highly smooth surface, which caused the film not to remain adhered to the surface.

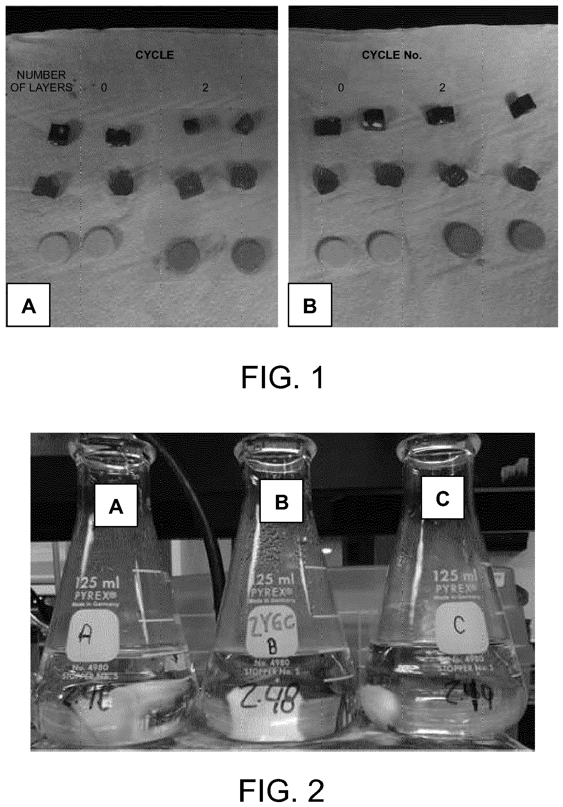

Figure 1 shows the different treatment systems with 0 and 2 coating layers. Panel A) shows the systems not subjected to thermal treatment (freezing/thawing cycle); in panel B) systems subjected to thermal treatment (freezing/thawing cycle) are observed.

For all test systems, after the 48-hour period, they underwent the *ex vivo* gastrointestinal simulator.

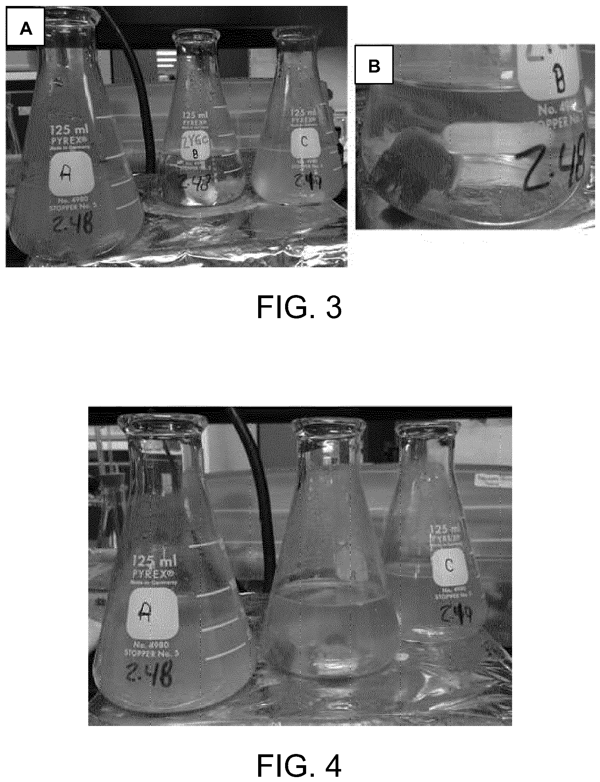

Later, the systems remained in constant stirring; in Figure 2A it is shown that there is a rapid solubility of the system, because the film coating them remained in the form of a hydrogel with a high water content, which in this form did not adhere to the surface, instead, in Figure 2B, the film remained adhered to the surface, because it was dehydrated (Figure 3).

After 20 minutes, the film managed to detach from the surface at B, as shown in Figure 4, but it is pointed out that the systems used were completely solubilized, whereas the film coating the systems still remained undissolved in the simulator.

For coating with PVA/pectin polymeric films a more porous and less smooth system is required on its surface, in order to obtain greater adhesion. This is why the use of sugar-coated gummies and tix-tix type candies was proposed, which have a rougher and more porous surface, respectively.

The same coating process was performed for both systems (zero and two layers) and under the same conditions in the gastrointestinal simulator (37 °C, pH ∼ 2.5), obtaining the following:
Within a few minutes, the film that coated system A began to separate from its surface. These results are probably due to the poor adhesion of the film to the surface of system A, which was still in the form of a hydrogel, instead, the film formed in B was totally adhered to the surface due to the formation of a flexible and free of moisture film, which caused high adhesion.

The adhesion of the film in B caused a greater protection to the solubility during the simulation process. After 4 minutes, an increase in the coloration of the solutions was observed, which indicates an increase in the solubility of the systems in the simulator.

The polymer films, dehydrated, formed from the polymeric composition of the invention showed greater adherence on the surface of the systems, which suggests greater protection against solubility in the gastrointestinal simulator.

### EXAMPLE 2

### Polymeric composition of PVA/Pectin

A mixture of polymers PVA (7.5 %) and pectin (5 %) was made. The PVA was added little by little in 100 ml of water at 80 °C, it was kept in constant stirring until its complete dissolution. The citrus pectin was added later, maintaining it in constant stirring at 80 °C until its dissolution.

### Preparation of a hydrogel

Once the polymeric mixture prepared according to what was previously described was cooled, it was poured into conical tube caps of 50 mL, used as molds for the hydrogel, then kept in freezing (-80 °C) for 6 days.

After the time elapsed, the hydrogels were carefully drilled with a spatula to place the red carmine dye into them in an amount of 100, 50 and 25 mg, then they were covered with the same PVA-PECTIN solution and they were frozen again for 2 days.

### Release profile of the carmine red dye in the hydrogel

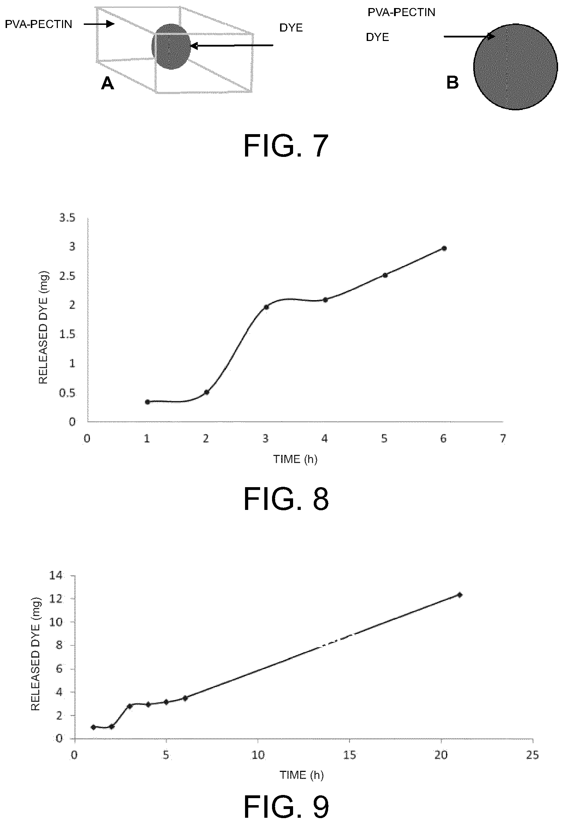

The release of the dye immersed in the gels was evaluated using an *ex vivo* gastrointestinal simulator, and according to the following two criteria:
1) Dye located inside the hydrogel in quantities of 25, 50, 100 mg for A, B and C respectively; and
2) Dye combined with the formulation of PVA-Pectin in quantities of 20 mg for each gel. (Figure 7)

The *ex vivo* gastrointestinal simulator in which the release evaluation was carried out consists of 5 reactors corresponding to Stomach, Small Intestine and the three sections of the Colon (Ascending, Transverse and Descending). The system maintains physiological conditions (with the enzymes characteristic of each section and at the appropriate pH) and constant temperature at 37 °C.

The stomach medium was adjusted to an initial pH of 2 to 2.5, Pepsin was added and the hydrogels were left for two hours.

Later, the medium was adjusted to pH 5 - 5.5 (small intestine conditions), it was added Lipase, Pancreatin and porcine Bile (commercially available), with a stirring speed of 50 rpm, thus allowing the diffusion of red carmine dye contained in the hydrogels, the evaluation was maintained for 4 hours. (Figures 8 and 9).

The measurement of release of the dye from the hydrogels was carried out by colorimetric method employing ultraviolet-visible spectroscopy, comparing against a calibration curve of the carmine red dye elaborated *ex profeso.* Results showed that the hydrogels prepared by combining the dye with the PVA-Pectin formulation presented a faster release profile (Figure 8) than the hydrogels in cube form (Figure 9).

## Claims

1. A gelled polymeric composition for controlled release in the digestive tract comprising:
a. a natural polymer that is selected from the group of: polysaccharides such as pectin, fructans, chitosan, chitin, celluloses, starches, modified starches, amyloses, amylopectins, mannans, galactomannans, and arabinocylanes;
b. a synthetic polymer selected from the group of synthetic water-soluble polymers such as: polyethylene glycol, polyvinyl alcohol, polyvinyl pyrrolidone, polyacrylic acid, polyphosphates.

2. A polymeric composition according to claim 1 **characterized in that** the natural polymer is a pectin and in a percentage of 1-6 %.

3. A polymeric composition according to claim 1 **characterized in that** the synthetic polymer is polyvinyl alcohol of molecular weight 89,000 - 98,000 KDa and in a percentage of 5 - 15 %.

4. Use of the polymeric composition as described in any of claims 1 to 3 for the manufacture of a vehicle for the pharmaceutical industry.

5. Use of the polymeric composition as described claim 1 for the manufacture of a vehicle for the food industry.

6. Use of the polymeric composition as described in claim 1 for the manufacture of a film.

7. Use of the polymeric composition as described in claim 1 for the manufacture of a hydrogel.

8. Use of the polymeric composition as described in claim 1 for the manufacture of a gel.

9. Use of the polymeric composition as described in claim 1 for the manufacture of a polymeric conjugate.

10. Use of the polymeric composition as described in claim 1 for the manufacture of a coating.

11. Use of the polymeric composition as described in claim 1 for the incorporation of substances including: drugs, food additives, bioactive substances, nutrients, dyes, gels, capsules, granules, spheres, particles, nanoparticles and/or mixtures thereof.
